# EUROPEAN PATENT APPLICATION

(11) **EP 4 614 151 A1**
(43) Date of publication of application: **10.09.2025**
(21) Application number: 24382243.4
(22) Date of filing: 06.03.2024
(51) Int. Cl.: G01N 33/573, A61K 39/00, A61P 1/00, G01N 33/68

(54) **IN VITRO METHOD FOR PREDICTING THE RESPONSE OF A PATIENT SUFFERING FROM ULCERATIVE COLITIS TO A TREATMENT WITH ANTI- TNF ANTIBODIES**

(71) Applicant: Fundación Pública Galega Instituto de Investigación Sanitaria de Santiago de Compostela, 15706 Santiago de Compostela A Coruña (ES); Servizo Galego De Saúde, 15703 Santiago de Compostela, A Coruna (ES)
(72) Inventor: CONDE ARANDA, Javier, 15706 Santiago de Compostela (ES); BARREIRO DE ACOSTA, Manuel, 15706 Santiago de Compostela (ES)
(74) Representative: Hoffmann Eitle

(57) **Abstract**

The present invention refers to an *in vitro* method for predicting the response of a patient suffering from ulcerative colitis to a treatment with anti-tumor necrosis factor (TNF) antibodies.

## Description

### FIELD OF THE INVENTION

The present invention refers to the medical field. Particularly, the present invention refers to an *in vitro* method for predicting the response of a patient suffering from ulcerative colitis to a treatment with anti-tumor necrosis factor (TNF) antibodies.

### STATE OF THE ART

Inflammatory bowel disease (IBD), which mainly comprises Crohn's disease (CD) and ulcerative colitis (UC), is a group of immune-mediated disorders of the gastrointestinal (GI) tract characterised by chronic and recurring aberrant inflammatory responses in the intestinal mucosa. While UC is restricted to the colon and presents a continuous inflammation confined to the mucosal layer, CD is characterised by a discontinuous and transmural inflammation that can occur anywhere in the GI tract. IBD does not have a cure, and despite the current therapies, a proportion of patients suffer from relapses and continuous inflammation, which sometimes lead to surgical removal of parts of the intestine.

The aetiology of the disease is not entirely clear. It has been shown that certain genetic and environmental factors contribute to the development of this disease. Furthermore, it is commonly accepted that an uncontrolled immune response due to defects in the microbiota and the intestinal epithelial barrier induces a positive feedback loop that makes inflammation chronic. Based on this, it is not surprising that the most effective therapies for IBD target crucial elements involved in the inflammatory response, such as tumour necrosis factor-alpha (TNF-α), leukocyte trafficking, or JAK signalling. However, these therapies do not offer a cure; therefore, patients with IBD require long-term treatments with associated side effects, such as opportunistic infections, because of immunosuppression. Apart from the unquestionable benefits of biologic and small molecule therapies, it is relevant to note that not all patients respond efficiently to these treatments. For instance, 10-30% of IBD patients do not show primary response to anti-TNF therapy, and 20-40% of patients lose efficacy over time. As a result, the health systems make an enormous economic effort, and IBD patients are exposed to undesirable side effects with no impact on their quality of life. This situation led the scientific community to seek predictive anti-TNF therapy tools that would avoid the risk of irreversible tissue damage due to uncontrolled intestinal inflammation in non-responder patients.

Many studies have reported transcriptional, cellular, or microbiome-derived predictive biomarkers for anti-TNF response. Unfortunately, none of them have been implemented in daily clinical practice so far, making the search for reliable and easy-to-read biomarkers an unmet need.

Since there is an unmet medical need of finding reliable biomarkers able to predict the response to anti-TNF therapy, the present invention is focused on solving this problem and an innovative method for predicting the response of a patient suffering from ulcerative colitis to a treatment with anti-TNF therapy is herein provided.

### DESCRIPTION OF THE INVENTION

### Brief description of the invention

The present invention refers to an *in vitro* method for predicting the response of a patient suffering from ulcerative colitis to a treatment with anti-TNF therapy, preferably anti-TNF antibodies, which comprises assessing the level of expression of a matrix metalloproteinase enzyme (MMPs), or a combination thereof.

So, the special technical feature which confers unity of invention to the present invention is the use of MMPs for predicting the response of a patient suffering from ulcerative colitis to a treatment with anti-TNF antibodies. MMPs are a family of zinc-dependent endopeptidases responsible for extracellular matrix (ECM) remodelling, among other relevant physiological and pathological processes. No information is available on the association between MMPs and anti-TNF treatment response. It is not known either whether a colonic MMP transcriptional signature could predict therapy response or whether these results extrapolate to circulating PBMCs.

The inventors of the present invention performed a meta-analysis of publicly available microarray and RNAseq datasets that resulted in the isolation of a strong and specific colonic transcriptional signature capable of predicting anti-TNF therapy response at baseline. Moreover, the inventors of the present invention found that the expression levels of specific MMPs were significantly upregulated in peripheral blood mononuclear cells (PBMCs) of non-responder patients. Altogether, the results show unique and easy-to-read candidates for anti-TNF therapy response prediction.

Particularly, the inventors of the present invention isolated a signature of 12 altered MMPs in human biopsies and blood samples active patients compared to healthy controls. Moreover, these MMPs show a different expression pattern between infliximab (IFX) responder patients and IFX non-responders. They were MME, MMP2, MMP3, MMP9, MMP10, MMP12, MMP19, AGTPBP1, MEP1A, HTRA1, PHEX and ATP23. Interestingly, to avoid the use of invasive techniques such as colonoscopy, blood samples were recruited to obtain the mRNA from peripheral blood mononuclear cells (PBMCs) of patients with moderate to severe Ulcerative Colitis (UC) before starting their relevant treatment. Then, the expression of these genes was analysed by RT-qPCR with samples obtained from our cohort of patients (n=17). In this case, a significant difference in membrane metalloendopeptidase (MME) expression levels were observed, according to the results analysed by bioinformatics. Of note, a different pattern was not seen in response to patients treated with vedolizumab (VDZ). Newly, this leads us to believe that MME could be a possible specific and predictive biomarker of response to anti-TNF therapy in PBMCs.

So, the first embodiment of the present invention refers to an *in vitro* method for predicting the response of a patient suffering from ulcerative colitis to a treatment with anti-TNF antibodies which comprises assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment, or ii) if the level of expression of the metalloproteinase enzyme is lower than a pre-established threshold value, it is an indication that the patient will respond to the treatment.

The second embodiment of the present invention refers to an *in vitro* method for deciding or recommending whether to treat a patient suffering from ulcerative colitis with anti-TNF antibodies which comprises assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment and the treatment with anti-TNF antibodies is not recommended, or ii) if the level of expression of the metalloproteinase enzyme is lower than a pre-established threshold value, it is an indication that the patient will respond to the treatment and a treatment with anti-TNF antibodies is recommended.

The third embodiment of the present invention refers to an *in vitro* method for classifying a patient suffering from inflammatory bowel disease into responder or non-responder patient to anti-TNF antibodies which comprises determining the level of expression of metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment and is classified as non-responder, or ii) if the level of expression of the metalloproteinase enzyme is lower than a pre-established threshold value, it is an indication that the patient will respond to the treatment and is classified as responder.

The fourth embodiment of the present invention refers to the *in vitro* use of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, for predicting the response of a patient suffering from ulcerative colitis to a treatment with anti-TNF antibodies, for deciding or recommending whether to treat a patient suffering from ulcerative colitis with anti-TNF antibodies, or for classifying a patient suffering from ulcerative colitis into responder or non-responder to anti-TNF antibodies.

The fifth embodiment of the present invention refers to the *in vitro* use of a kit consisting of reagents for measuring the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, for predicting the response of a patient suffering from ulcerative colitis to a treatment with anti-TNF antibodies, for deciding or recommending whether to treat a patient suffering from ulcerative colitis with anti-TNF antibodies, or for classifying a patient suffering from ulcerative colitis into responder or non-responder to anti-TNF antibodies.

The sixth embodiment of the present invention refers to anti-TNF antibodies, or any pharmaceutical composition comprising thereof, optionally including pharmaceutically acceptable excipients or carriers, for use in the treatment of a patient suffering from ulcerative colitis, wherein method comprises classifying the patient as a responder to anti-TNF antibodies by assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment, or ii) if the level of expression of the metalloproteinase enzyme is lower than a pre-established threshold value, it is an indication that the patient will respond to the treatment.

Alternatively, the present invention refers to a method for treating patients suffering from ulcerative colitis which comprises administering to the patient a pharmaceutically effective amount of anti-TNF antibodies; wherein method comprises classifying the patient as a responder to anti-TNF antibodies by assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment, or ii) if the level of expression of the metalloproteinase enzyme is lower than a pre-established threshold value, it is an indication that the patient will respond to the treatment.

In a preferred embodiment, the present invention compromises assessing the level of expression of the following combination of metalloproteinase enzymes: MME, MMP2, MMP3 and PHEX wherein: i) if the level of expression of the metalloproteinase enzymes is higher than a pre-established threshold value, it is an indication that the patient will not respond to the treatment and the treatment with anti-TNF antibodies is not recommended, or ii) if the level of expression of the metalloproteinase enzymes is lower than a pre-established threshold value, it is an indication that the patient will respond to the treatment and a treatment with anti-TNF antibodies is recommended.

In a preferred embodiment, the anti-TNF is a monoclonal antibody selected from: infliximab, adalimumab, certolizumab pegol, and/or golimumab.

In a preferred embodiment, the biological sample is selected from the group comprising: colon tissue, blood, serum, plasma or peripheral blood mononuclear cells (PBMCs) isolated from peripheral blood.

Alternatively, the present invention refers to:
A method for identifying or detecting biomarker signatures for predicting the response of a patient suffering from ulcerative colitis to a treatment with anti-TNF antibodies, which comprises assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment, or ii) if the level of expression of the metalloproteinase enzyme is lower than a pre-established threshold value, it is an indication that the patient will respond to the treatment

In a preferred embodiment, the present invention is a computer-implemented invention, wherein a processing unit (hardware) and a software are configured to: a) Receive level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, b) process the level of expression for finding substantial variations or deviations, and c) provide an output through a terminal display of the variation or deviation of the level of expression, wherein the variation or deviation of the level of expression is indicative of whether a patient suffering from ulcerative colitis to a treatment with anti-TNF antibodies.

For the purpose of the present invention, the following terms are defined:
- The term "comprising" means "including", but not limited to what follows the term "comprising". Thus, the use of the term "comprising" indicates that the elements listed are necessary or mandatory, but that other elements are optional and may or may not be present.
- The term "consisting of" means "including" but is limited to what follows the term "consisting of". Thus, the term "consists of" indicates that the elements listed are mandatory, and that other elements may not be present.
- By "therapeutically effective dose or amount" of a composition comprising anti-TNF antibodies is intended an amount that, when administered, brings about a positive therapeutic response in a subject having ulcerative colitis. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Brief description of the figures

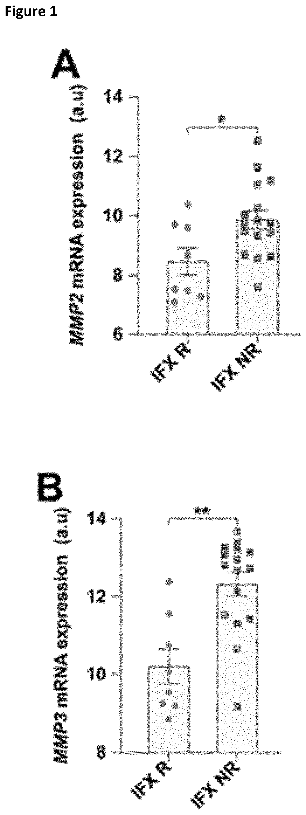

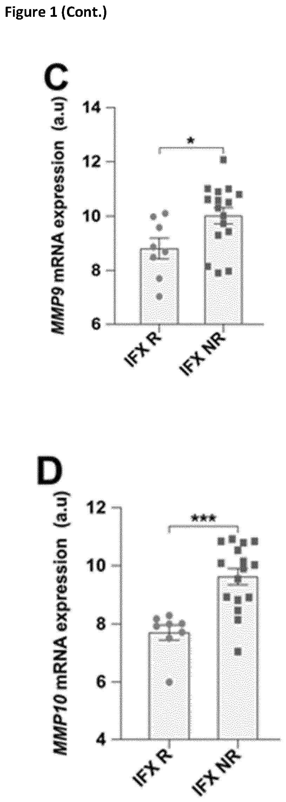

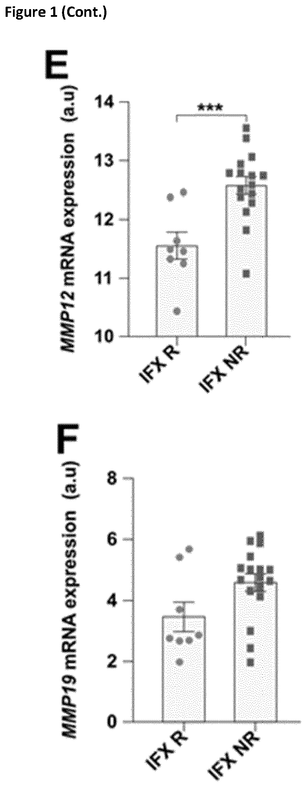

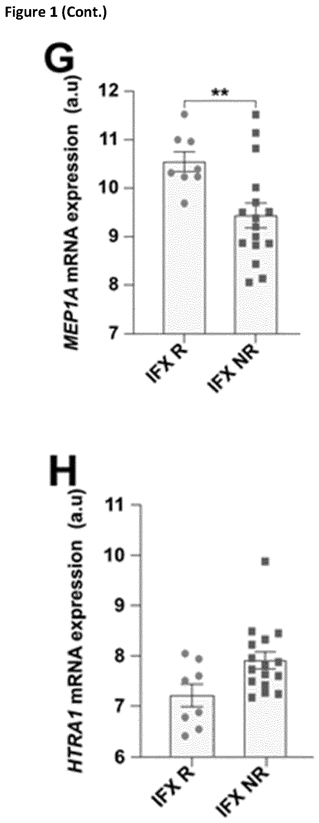

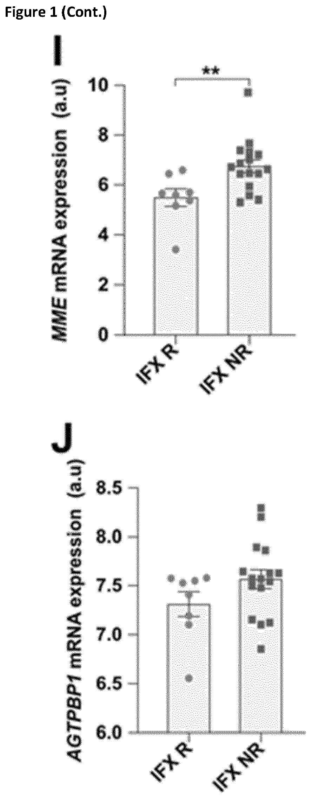

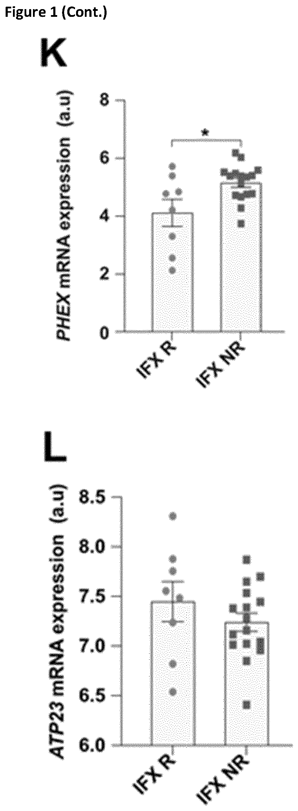

**Figure 1****. The expression of a selection of MMPs is dysregulated in nonresponder patients to anti-TNF therapy. (A-L)** Relative expression of the interrogated transcripts in colon biopsies from the indicated groups of patients. The results were retrieved from the GEO microarray dataset GSE16879. IFX R=Infliximab responder; IFX NR=Infliximab nonresponder. *, P ≤ 0.05; **, P ≤ 0.01; ***, P ≤ 0.001 relative to IFX R.

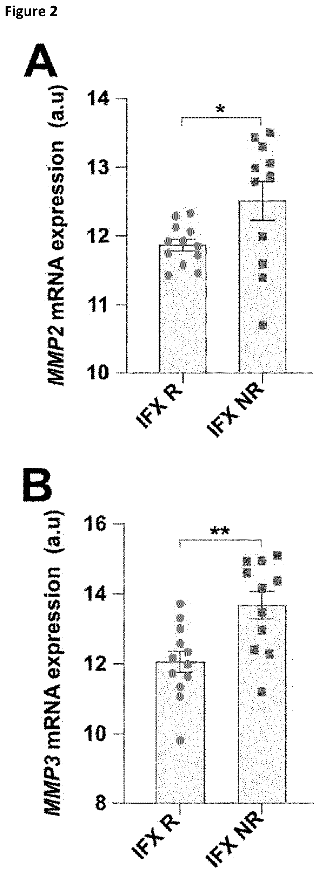

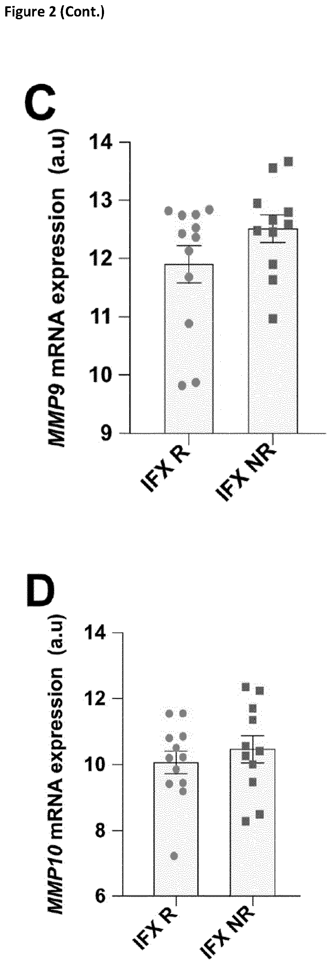

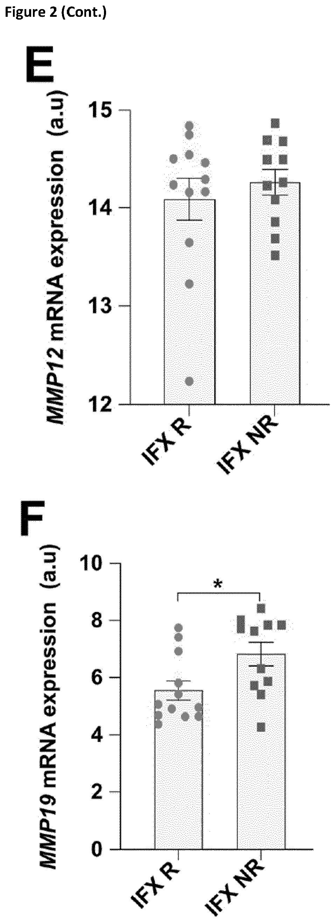

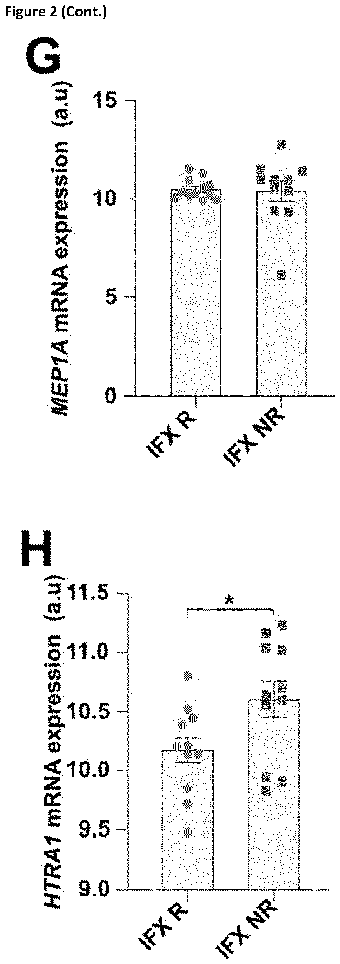

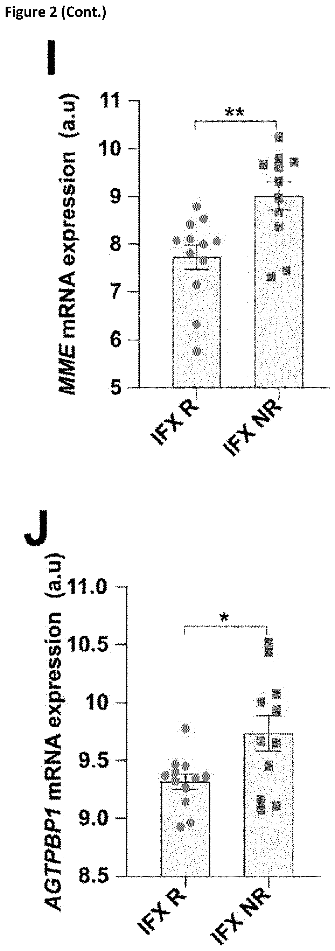

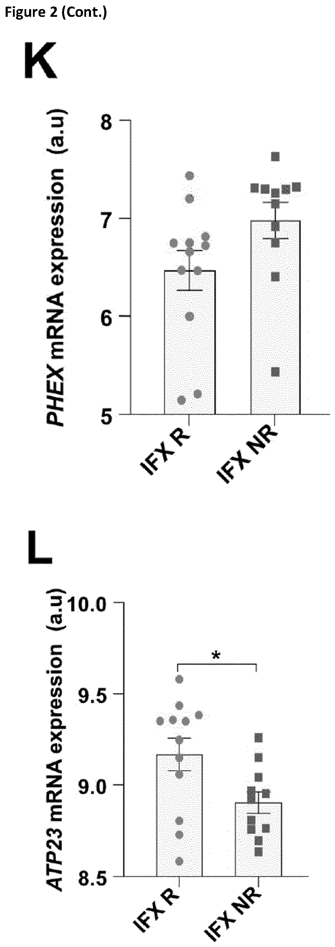

**Figure 2****. Corroboration of the altered expression of a selection of MMPs in nonresponder patients to anti-TNF therapy. (A-L)** Relative expression of the interrogated transcripts in colon biopsies from the indicated groups of patients. The results were retrieved from the GEO microarray dataset GSE12251. IFX R=Infliximab responder; IFX NR=Infliximab nonresponder. *, P ≤ 0.05; **, P ≤ 0.01 relative to IFX R.

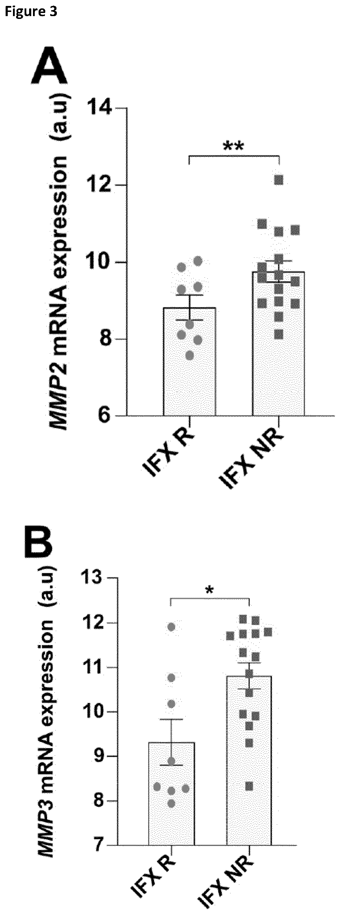

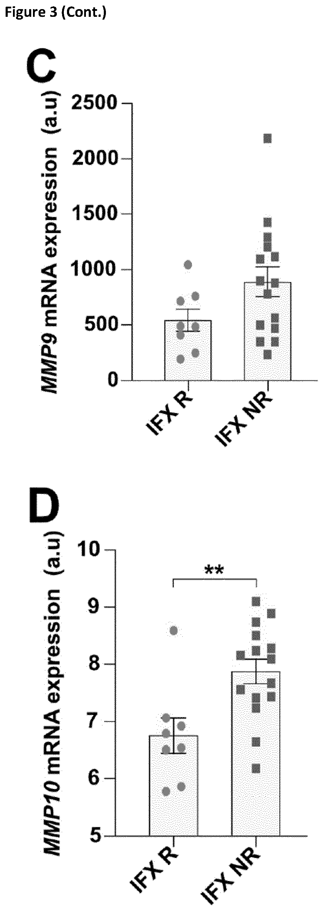

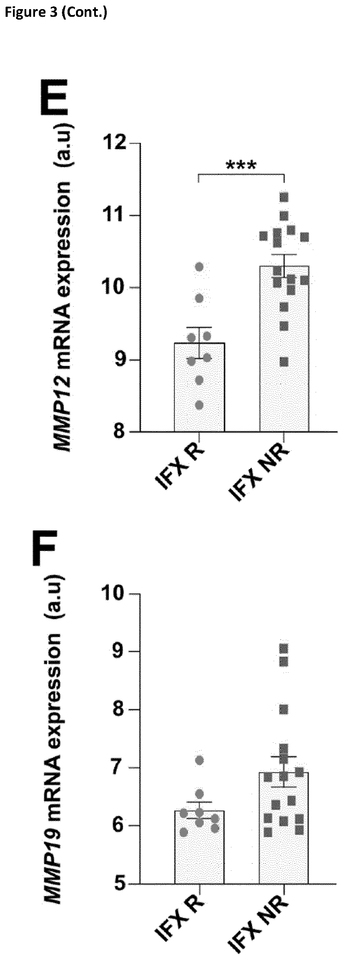

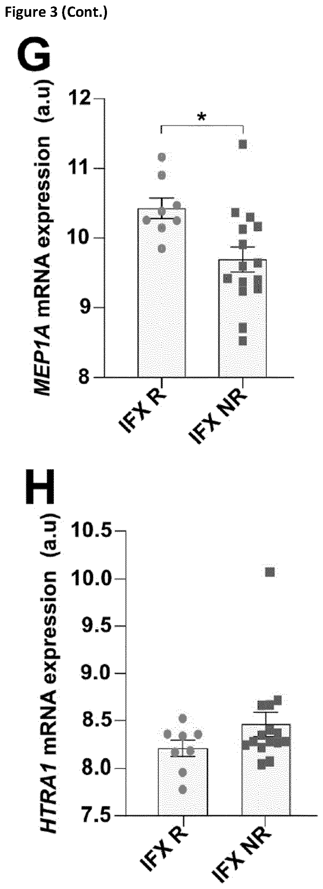

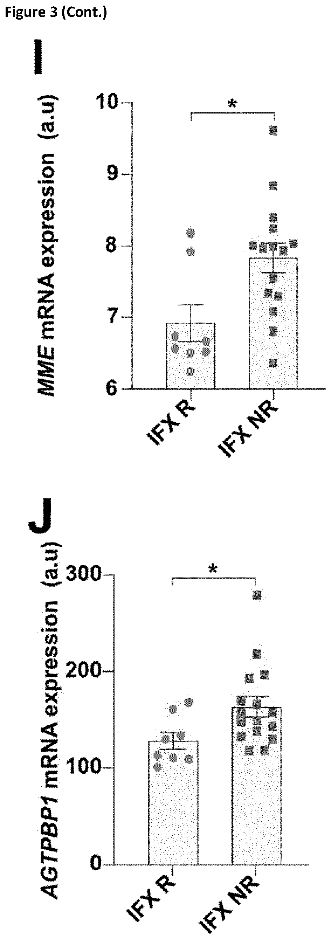

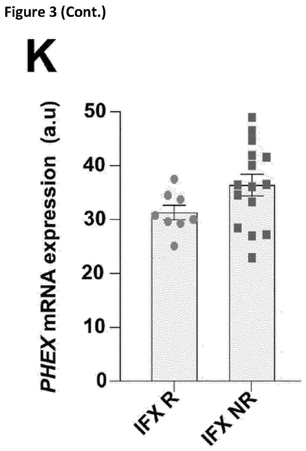

**Figure 3****. Confirmation of the altered expression of a selection of MMPs in nonresponder patients to anti-TNF therapy. (A-K)** Relative expression of the interrogated transcripts in colon biopsies from the indicated groups of patients. The results were retrieved from the GEO microarray dataset GSE73661. IFX R=Infliximab responder; IFX NR=Infliximab nonresponder. *, P ≤ 0.05; **, P ≤ 0.01; ***, P ≤ 0.001 relative to IFX R.

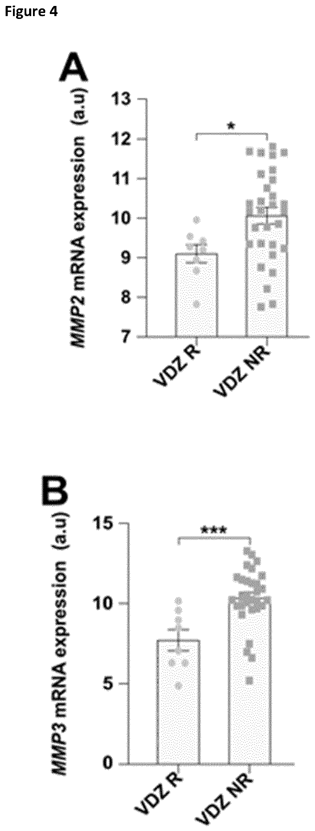

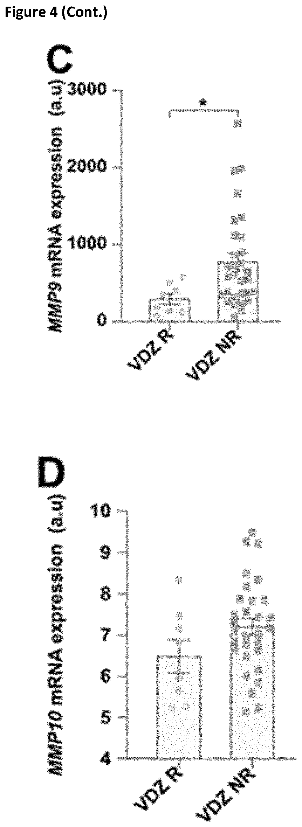

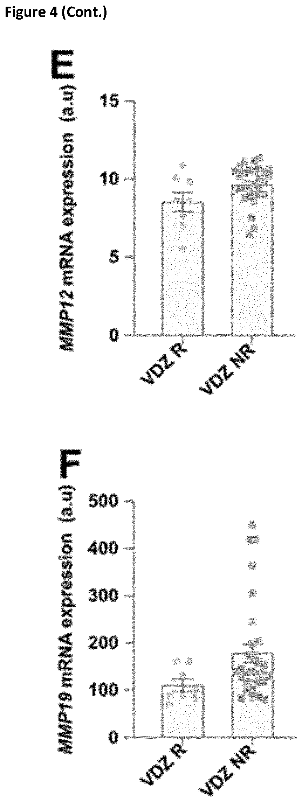

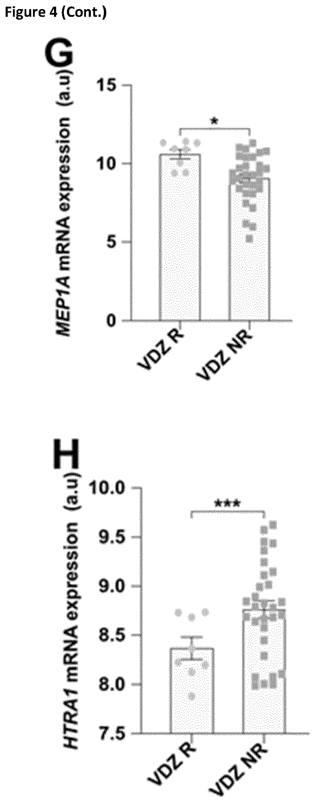

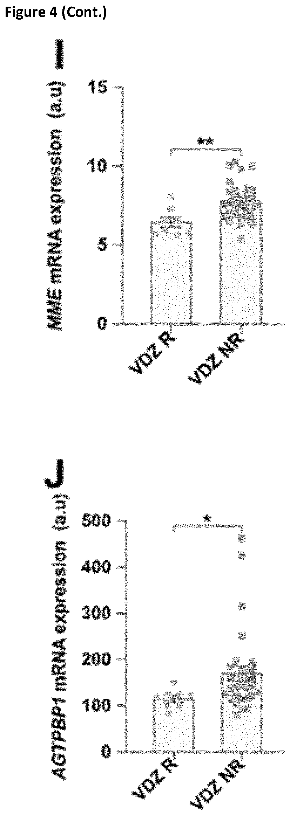

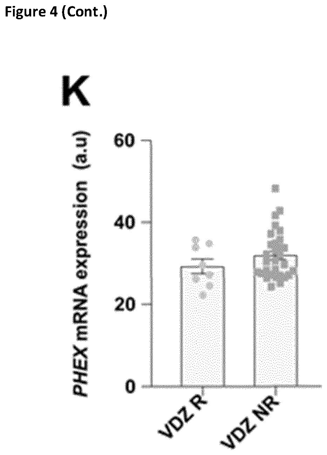

**Figure 4****. The expression of a selection of MMPs is less dysregulated in nonresponder patients to vedolizumab therapy. (A-K)** Relative expression of the interrogated transcripts in colon biopsies from the indicated groups of patients. The results were retrieved from the GEO microarray dataset GSE73661. VDZ R=vedolizumab responder; VDZ NR=vedolizumab nonresponder. *, P ≤ 0.05; **, P ≤ 0.01; ***, P ≤ 0.001 relative to VDZ R.

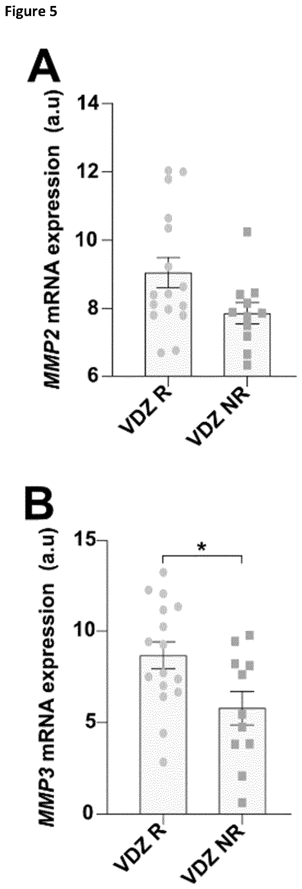

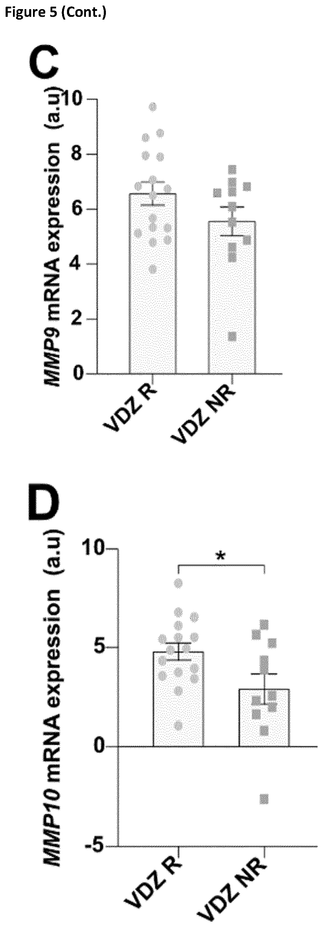

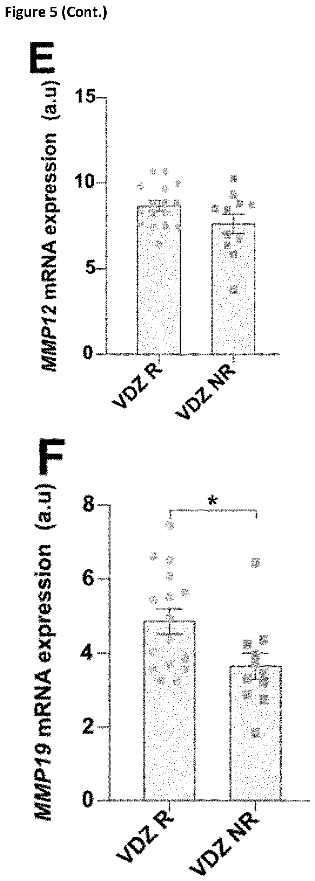

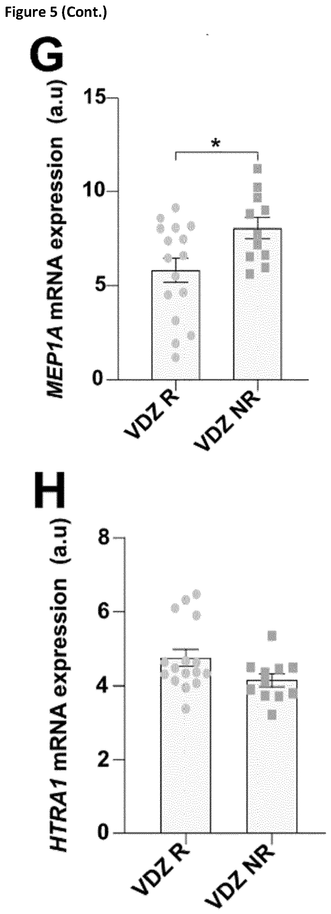

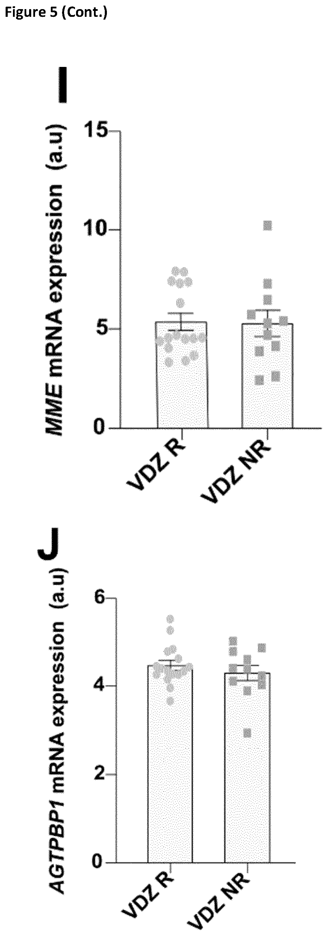

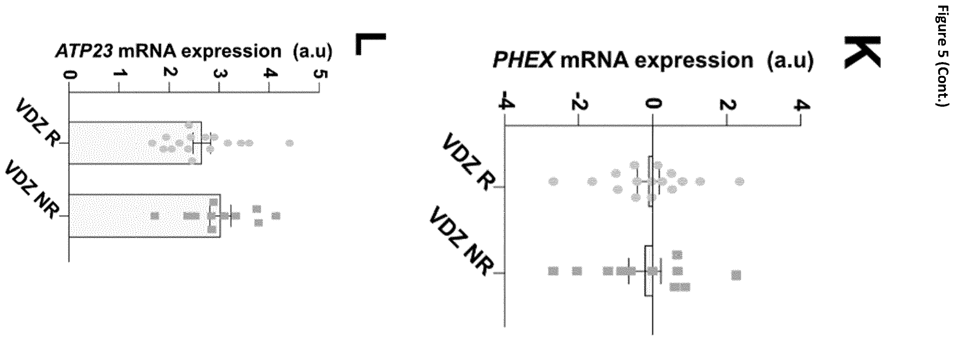

**Figure 5****. The expression of a selection of MMPs is not dysregulated in nonresponder patients to vedolizumab therapy. (A-L)** Relative expression of the interrogated transcripts in colon biopsies from the indicated groups of patients. The results were retrieved from the RNAseq dataset E-MTAB-7845. VDZ R=vedolizumab responder; VDZ NR=vedolizumab nonresponder. *, P ≤ 0.05 relative to VDZ R.

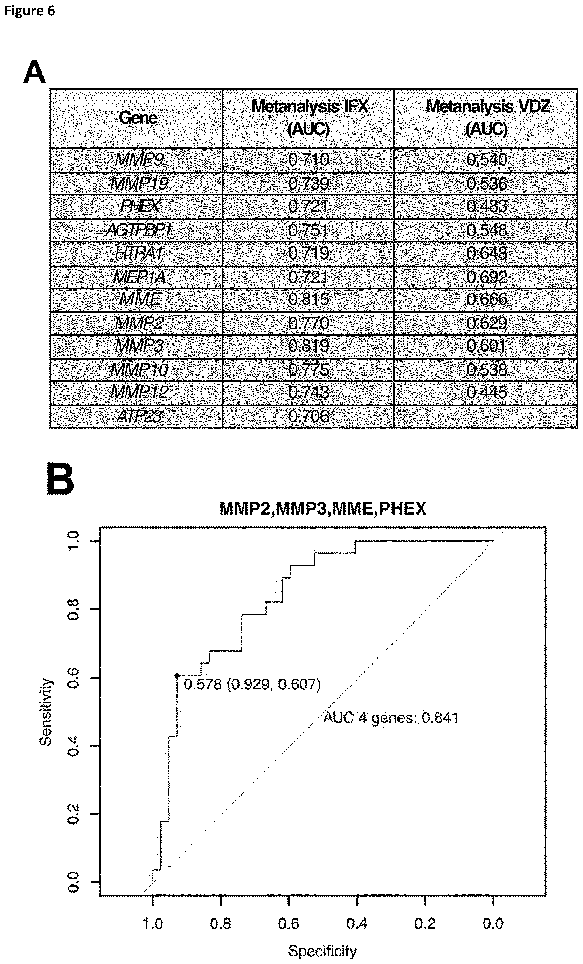

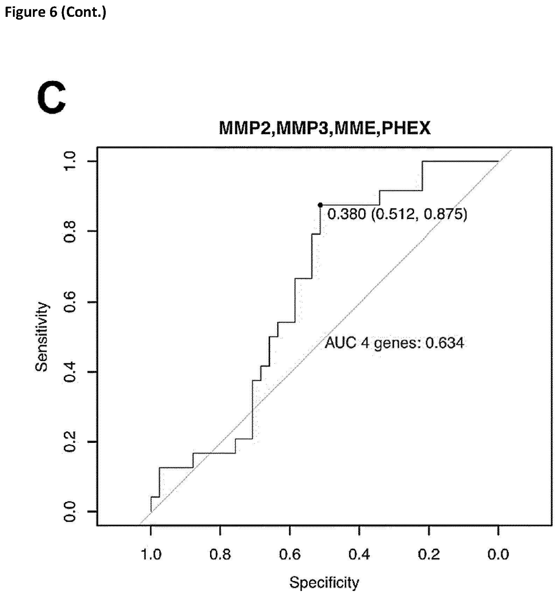

**Figure 6****. A four-gene MMP transcriptional signature predicts the response to anti-TNF therapy. (A)** Summary of ROC analysis of expression of the interrogated transcripts in colon biopsies metanalysis from anti-TNF (GSE16879; GSE73661; GSE12251) and vedolizumab (GSE7366; E-MTAB-7845) responder and nonresponder patients. **(B)** ROC analysis of expression of the indicated MMP signature in colon biopsies from anti-TNF responder and nonresponder patients of combined GEO microarray datasets GSE16879; GSE73661; GSE12251. **(C)** ROC analysis of expression of the indicated MMP signature in colon biopsies from vedolizumab responder and nonresponder patients of combined GEO microarray dataset GSE73661 and RNAseq dataset E-MTAB-7845.

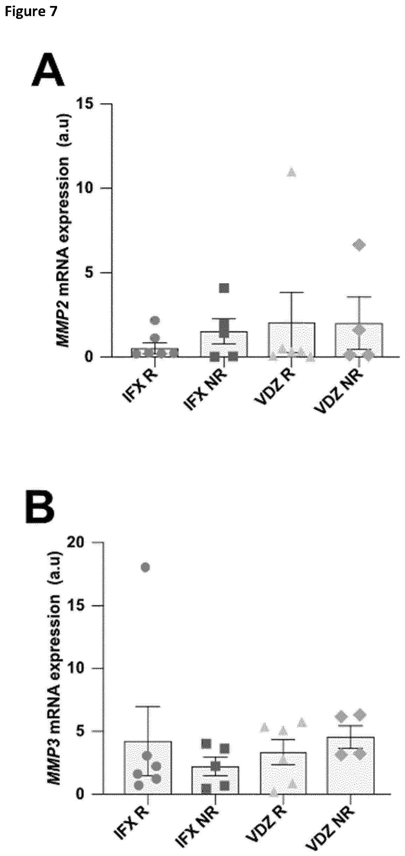

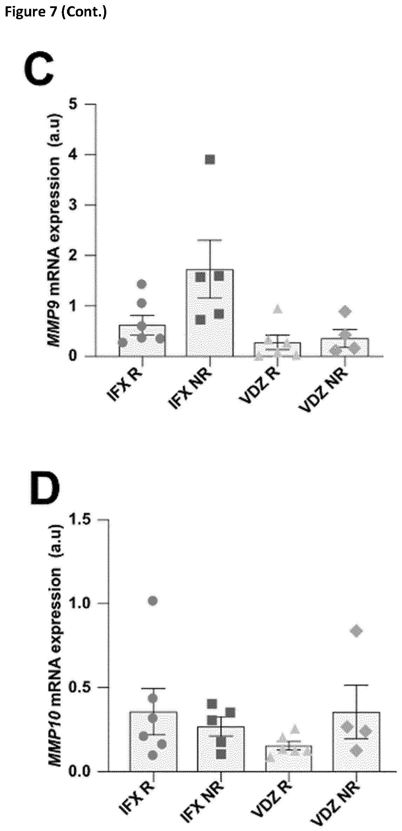

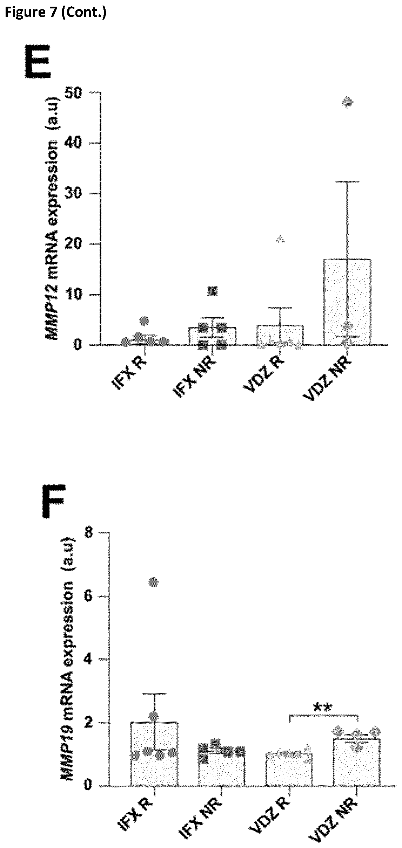

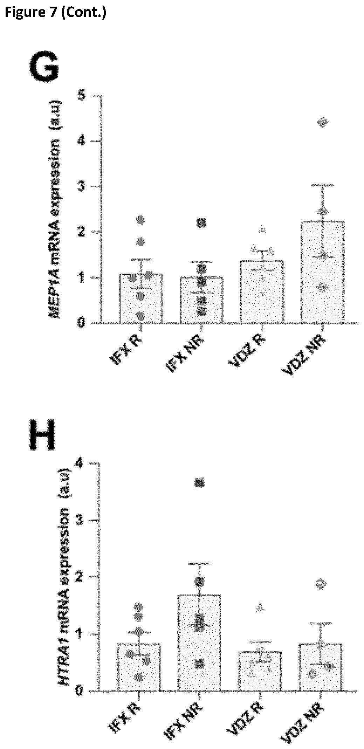

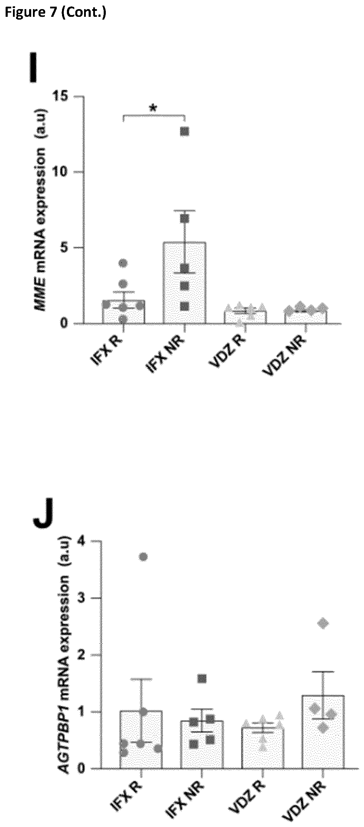

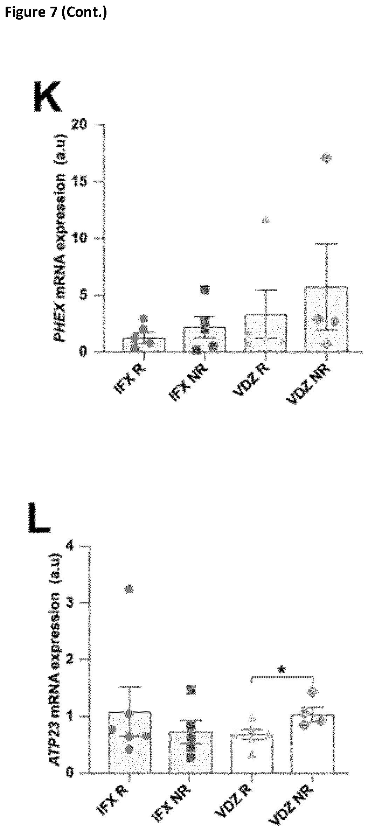

**Figure 7****. The expression of MME is upregulated in PBMCs from nonresponder patients to anti-TNF therapy. (A-L)** Relative mRNA expression of the interrogated transcripts in PBMCs from the indicated groups of patients. IFX R=Infliximab responder; IFX NR=Infliximab nonresponder; VDZ R=vedolizumab responder; VDZ NR=vedolizumab nonresponder. *, P ≤ 0.05 relative to IFX R or VDZ R.

### Detailed description of the invention

The present invention is illustrated by means of the Examples set below without the intention of limiting its scope of protection.

### Example 1. MATERIAL AND METHODS

### Example 1.1. Bioinformatics of human array datasets

The National Center for Biotechnology Information (NCBI) at the National Library of Medicine (NLM) supports the Gene Expression Omnibus (GEO), a database that accepts raw and processed data with written descriptions of experimental design, sample characteristics, and methodology for studies of high-throughput gene expression and genomics. GEO accession codes for other datasets used in this work include GSE38713 (human colonic biopsies), GSE59071 (human colonic biopsies), GSE16879 (human colonic biopsies), GSE12251 (human colonic biopsies), GSE73661 (human colonic biopsies), E-MTAB-7845 (human colonic biopsies) and GSE191328 (blood samples).

### Example 1.2. Patients

Twenty-two active ulcerative colitis moderate-to-severe patients according to the European Crohn's and Colitis Organisation criteria have been recruited from the Inflammatory Bowel Disease Unit of the Santiago University Clinical Hospital. Blood samples were obtained at initial time (T0) before starting the relevant treatment. All patients were informed of the procedure and signed the corresponding informed consent form. All protocols performed on patients were approved by the "Comité de ética de la investigación con medicamentos de Galicia" registration code 2020/357. Monitoring of patient treatment is carried out using markers that provide information on the state of inflammation, such as C-reactive protein (CRP), calprotectin and the Mayo index. Thanks to these markers, it is possible to know the response of patients to the biological drug, differentiating between responders and non-responders.

### Example 1.3. Isolation of peripheral blood mononuclear cells (PBMCs)

Peripheral blood was collected in sodium heparin tubes and 1: 1 blood/PBS (1X) mixture was slowly added to Ficoll-Paque Plus (Sigma-Aldrich, MO, USA) and then centrifuged at 2000 rpm, 30 minutes, without brake to obtain a density gradient. PBMCs were collected from the interphase between the Ficoll-Paque Plus and the plasma. Polymorphonuclear cells (PMNs) were then obtained by the precipitation of erythrocytes with dextran (Thermo Fisher Scientific, MA, USA) 3% in 0.9% NaCl and several washes with ACK buffer.

### Example 1.4. RNA isolation

Total RNA was extracted from PBMCs and PMNs with NZYol (NZYTech, Portugal) and E.Z.N.A Total RNA Kit (Omega Bio-tek, GA, USA) according to the manufacturer's instructions. Total RNA quantity and quality was assessed using the NanoDrop ND-1000 spectrophotometer (NanoDrop Technologies, Wilmington, DE). Only samples with an RNA integrity number (RIN) greater than 7.0 were used.

### Example 1.5. Real-time reverse transcription-polymerase chain reaction (RT-qPCR)

The reverse transcription quantitative real-time polymerase chain reaction (RT-qPCR) was performed using a Power SYBR^{™} Green RNA-to-CT^{™} 1-Step Kit (Applied Biosystems, MA, USA). The data were calculated, using the comparative (ΔΔCt) method and the Quantstudio Design and Analysis Software v. 1.5.2 (Quantstudio) (Applied Biosystems, MA, USA). Note that the gene expression levels were normalized to β-actin. Melting curves were generated to ensure a single gene-specific peak, and no-template controls were included for each run and each set of primers to control for unspecific amplifications.

### Example 1.6. Statistical analysis

Statistical analysis was performed by using the GraphPad Prism 8.0.1 software (California, USA). Quantitative data were presented as mean ± standard error of the mean (SEM). Two-group comparisons were performed using a two-tailed Student's t-test. Multi group comparisons were performed using one-way ANOVA followed Bonferroni multiple comparisons test. A P value < 0.05 was considered statistically significant.

### Example 2. RESULTS

### Example 2.1. Several MMPs are associated with UC activity

First, we analysed the colonic expression levels of thirty-two MMP-related genes, retrieved from the Kyoto Encyclopedia of Genes and Genomes (KEGG) database, in two independent microarray datasets. Many MMPs displayed similar expression levels or inconsistencies among the indicated groups analysed or datasets. However, 12 out of 32 genes presented a robust dysregulated expression pattern in active UC patients compared with inactive and remission or non-IBD controls. These twelve most dysregulated genes were separately analysed, and MMP2, MMP3, MMP10, MMP12, HTRA1, MMP9, MMP19, AGTPBP1, PHEX and MME transcripts were found to be significantly increased in patients with active disease. In contrast, MEP1A and ATP23 expression levels were upregulated in healthy, UC inactive, or UC remission colonic samples compared with UC patients with active disease. We selected these twelve genes for further analysis on the basis of their observed alteration according to the clinical status.

### Example 2.2. MME, MMP2, MMP3, MMP10, MMP12, HTRA1, MMP9, MMP19, AGTPBP1, PHEX, MEP1A, and ATP23 colonic expression is associated with anti-TNF response at baseline

Next, we analysed the expression levels of the twelve selected MMPs in three independent cohorts of UC patients starting anti-TNF therapy. Interestingly, we observed that most of the MMPs that were upregulated in the colonic samples of active UC patients showed increased mRNA expression in samples collected at baseline from non-responder UC patients to anti-TNF therapy (**Figure 1****,** **2** and **3**). In contrast, the expression of MEP1A and ATP23 was found to be decreased in the same conditions (**Figure 1****,** **2** and **3**).

To determine whether the observed MMP expression pattern was specific to anti-TNF response and not a common feature associated with other biologic treatments or the clinical status, we performed the same analysis in colon biopsy samples obtained at baseline in patients starting vedolizumab therapy. Although some of the genes analysed in one cohort of vedolizumab responder and non-responder UC patients presented a behaviour similar to that observed for anti-TNF-treated patients, MMP10, MMP12, MMP19 and PHEX did not differ significantly between the two groups of patients starting vedolizumab treatment (**Figure 4**). To note, we tested those results in a second independent cohort, which revealed that most of the MMP transcripts presented similar expression levels regardless of the response to vedolizumab (**Figure 5**). Only MMP3, MMP10, MEP1A and MMP19 showed statistically significant differences in vedolizumab non-responder patients. However, the observed pattern was opposite to the described for patients treated with infliximab (**Figure 5**).

### Example 2.3. MMP signature predicts the responsiveness to anti-TNF therapy

We studied in detail the performance of the selected twelve MMPs, at the colon level, in predicting the response to infliximab in the former three independent cohorts of infliximab-treated patients and two cohorts of vedolizumab-treated patients. In order to test the strength of our model, we performed a metanalysis using the same above databases. We found medium-high anti-TNF prediction power of all MMPs analysed, especially MMP3 and MME (ROC AUC = 81%). Interestingly, those MMPs showed significantly reduced predictive capability in pre-treated vedolizumab patients (ROC AUC between 60-66%) (**Figure 6A**), suggesting the specificity of these colonic MMP transcripts for discriminating anti-TNF responders from non-responders.

Next, we explored whether combining MMPs could result in better anti-TNF response prediction. As observed in **Figure 6B****,** grouping MMP2, MMP3, MME and PHEX improved the isolation of patients in which anti-TNF therapy will fail (ROC AUC = 84%). It is noteworthy that the colonic expression of that transcriptional signature was not capable of predicting vedolizumab response (ROC AUC = 63%) (**Figure 6C**).

### Example 2.4. MME is significantly increased in PBMCs from anti-TNF non-responder patients

Any useful biomarker should be characterised by its easy monitorisation. Although most patients with UC are subjected to a routine colonoscopy before starting biologic therapy, and the isolation of colon tissue and analysis of the previously described transcriptional signature would be feasible. The detection of predictive markers using less invasive techniques is advantageous. Therefore, we performed RT-qPCR studies in PBMCs from our local cohort of UC patients **(****Figure 7**). Interestingly, from all twelve MMPs analysed, we found a statistically significant increase in the baseline expression of MME in non-responder patients to anti-TNF therapy, which was not observed in pre-treated vedolizumab patients (**Figure 7I**). MMP9 expression showed the same tendency as MME(**Figure 7C**).

## Claims

1. *In vitro* method for predicting the response of a patient suffering from ulcerative colitis to a treatment with anti-tumor necrosis factor (TNF) antibodies which comprises assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment, or ii) if the level of expression of the metalloproteinase enzyme is lower than a pre-established threshold value, it is an indication that the patient will respond to the treatment.

2. *In vitro* method for deciding or recommending whether to treat a patient suffering from ulcerative colitis with anti-TNF antibodies which comprises assessing the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment and the treatment with anti-TNF antibodies is not recommended, or ii) if the level of expression of the metalloproteinase enzyme is lower than a pre-established threshold value, it is an indication that the patient will respond to the treatment and a treatment with anti-TNF antibodies is recommended.

3. *In vitro* method for classifying a patient suffering from inflammatory bowel disease into responder or non-responder patient to anti-TNF antibodies which comprises determining the level of expression of metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, in a biological sample obtained from the patient, wherein: i) if the level of expression of the metalloproteinase enzyme is higher than a pre-established threshold value, it is an indication that the patient will not respond to the treatment and is classified as non-responder, or ii) if the level of expression of the metalloproteinase enzyme is lower than a pre-established threshold value, it is an indication that the patient will respond to the treatment and is classified as responder.

4. *In vitro* method, according to any of the previous claims, which comprises assessing the level of expression of the following combination of metalloproteinase enzymes: MME, MMP2, MMP3 and PHEX.

5. *In vitro* method, according to any of the previous claims, wherein the anti-TNF is a monoclonal antibody selected from: infliximab, adalimumab, certolizumab pegol, and/or golimumab.

6. *In vitro* method, according to any of the previous claims, wherein the biological sample is selected from the group comprising: colon tissue, blood, serum, plasma or peripheral blood mononuclear cells (PBMCs) isolated from peripheral blood.

7. *In vitro* use of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, for predicting the response of a patient suffering from ulcerative colitis to a treatment with anti-TNF antibodies, for deciding or recommending whether to treat a patient suffering from ulcerative colitis with anti-TNF antibodies, or for classifying a patient suffering from ulcerative colitis into responder or non-responder to anti-TNF antibodies.

8. *In vitro* use of a kit consisting of reagents for measuring the level of expression of a metalloproteinase enzyme selected from the group consisting of: MME, MMP3, PHEX, MMP9, MMP10, AGTPBP1, MMP2, MMP19, MMP12, MEP1A, HTRA1 and/or ATP23; or any combination thereof comprising from 2 to 12 biomarkers, for predicting the response of a patient suffering from ulcerative colitis to a treatment with anti-TNF antibodies, for deciding or recommending whether to treat a patient suffering from ulcerative colitis with anti-TNF antibodies, or for classifying a patient suffering from ulcerative colitis into responder or non-responder to anti-TNF antibodies.

9. *In vitro* use, according to any of the claims 8 or 9, of the following combination of metalloproteinase enzymes: MME, MMP2, MMP3 and PHEX.

10. *In vitro* use, according to any of the claims 8 to 10, wherein the anti-TNF is a monoclonal antibody selected from: infliximab, adalimumab, certolizumab pegol, and/or golimumab.

11. *In vitro* use, according to any of the claims 8 to 12, wherein the biological sample is selected from the group comprising: colon tissue, blood, serum, plasma or peripheral blood mononuclear cells (PBMCs) isolated from peripheral blood.

12. Anti-TNF antibody, or any pharmaceutical composition comprising thereof, optionally including pharmaceutically acceptable excipients or carriers, for use in the treatment of a patient suffering from ulcerative colitis, wherein method comprises classifying the patient as a responder to anti-TNF antibodies, by following the method of the claims 1 a 6.

13. Anti-TNF antibody for use, according to claim 12, **characterized in that** it is a monoclonal antibody selected from: infliximab, adalimumab, certolizumab pegol, and/or golimumab.
